# EUROPEAN PATENT APPLICATION

(11) **EP 1 655 054 A1**
(43) Date of publication of application: **10.05.2006**
(21) Application number: 05023119.0
(22) Date of filing: 24.10.2005
(51) Int. Cl.: A61N 5/06, A61N 1/36, A61M 21/00

(54) **Neurosensory stimulation device**

(30) Priority: 28.10.2004 IT BO20040672
(71) Applicant: Montecorboli, Umberto, 29010 Pontenure PC (IT)
(72) Inventor: Montecorboli, Umberto, 29010 Pontenure PC (IT)
(74) Representative: Fanzini, Valeriano

(57) **Abstract**

A device for neurosensory stimulation of a user (2) comprises emitter means (3, 4, 5, 6, 7, 8) for a neurosensory stimulation signal which can be perceived by a user (2), and means (14) for modulating the signal emitted.

## Description

The present invention relates to a device for neurosensory stimulation of a user.

It is known that when a person lives through an event that is stressful at a physical or psychological level, the normal process of memory consolidation may become dysfunctional to the point that it may turn into a kind of pathology.

This pathology, known as P.T.S.D. (Post Traumatic Stress Disorder), is characterised by significant symptoms such as obsessive thoughts, sleep disturbances, poor concentration, sudden flashbacks, emotional stress.

This may occur in persons who experience traumatic events first hand or who witness them, involving the perception of grave danger to their physical safety or their life and associated with fear, horror and a sense of powerlessness. Such events may be natural (for example earthquakes, volcanic eruptions, floods, etc.) or caused by man (such as wars, serious accidents, torture, sexual abuse, etc.).

One method for treating patients affected by P.T.S.D. involves reducing their hypersensitivity to the trauma and guiding them in reprocessing it.

This result may be achieved using E.M.D.R. (Eye Movement Desensitisation and Reprocessing), a psychotherapeutic method perfected since 1989 by Dr. F. Shapiro.

Dr. Shapiro realised that alternate right/left eye movement stimulation could induce recovery of information stored anomalously in the neural networks and accelerate its correct reprocessing.

The type of stimulation initially used in E.M.D.R. was eye movement: the patient was asked to think about the trauma experienced again while following the movement of the therapist's hand only with his eyes.

The visual stimulus, obtained with light devices, is now used with auditory and tactile stimuli.

There are now devices for saccadic eye movement stimulation which emit visual signals. However, these devices have the disadvantage of being repetitive so that the user tends to become accustomed to them, meaning that on the whole they are not very effective if used as such.

Therefore, a neurosensory stimulation device was provided with the characteristics described in claim 1.

The present invention provides an improved stimulation device, more versatile and effective in a range of user conditions and such that it prevents the risks of the user becoming accustomed to it.

The present invention also relates to a neurosensory stimulation apparatus comprising a processing system connected to a device in accordance with the present invention.

The technical characteristics of the device according to the invention are clearly described in the claims below and its advantages are apparent from the detailed description which follows, with reference to the accompanying drawings which illustrate a preferred embodiment of the invention provided merely by way of example without restricting the scope of the inventive concept, and in which:

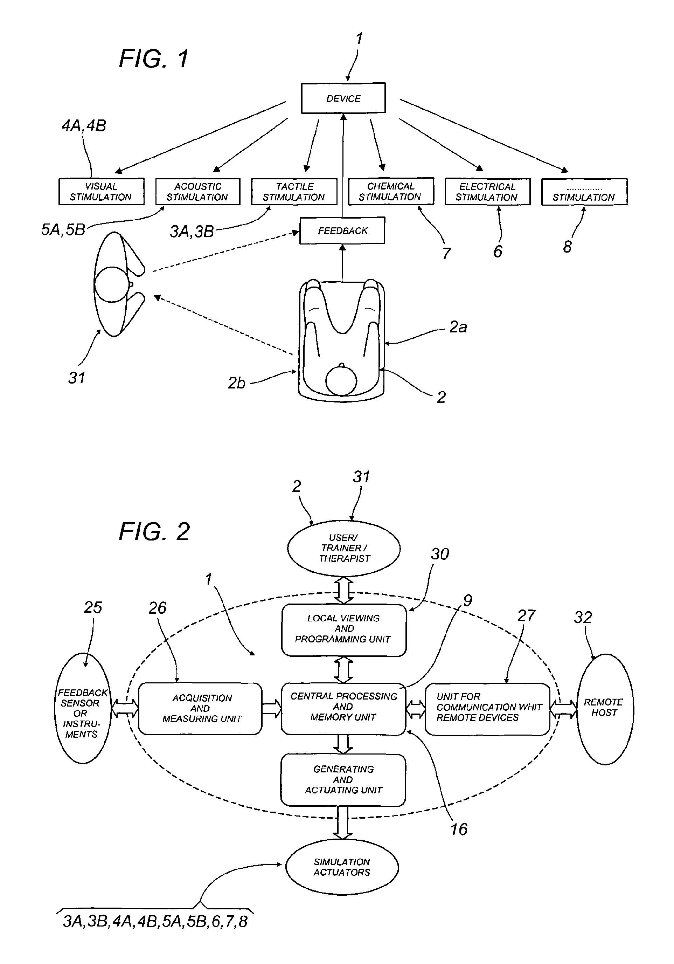

- Figure 1 is a top plan view, partly in blocks, of an application of the device in accordance with the present invention;
- Figure 2 is a block diagram of an apparatus for user neurosensory stimulation in accordance with the present invention;
- Figure 3 is a schematic perspective view, partly in blocks, of a device in accordance with the present invention.

With reference to the accompanying drawings, the numeral 1 denotes as a whole a device for user 2 neurosensory stimulation, in accordance with the present invention.

The device 1 comprises a series of emitter elements, or emitters, 3A, 3B, 4A, 4B, 5A, 5B, 6, 7 and 8 for a respective stimulation signal which can be perceived by the user 2.

The emitters 3A, 3B, 4A, 4B, 5A, 5B are of the same type and in pairs, that is to say, emitters 3A and 3B transform the respective signal so that it can be perceived by touch by the user, emitters 4A and 4B transform the respective signal so that it can be perceived visually, whilst emitters 5A and 5B transform the respective signal so that it can be perceived by the user 2 acoustically.

Advantageously, in embodiments not illustrated, the stimulation signal may be converted into an electrical signal 7, for example through a pair of electrodes which can be applied to the user's body, or into a suitable chemical or pharmacological stimulus 6 or other type of stimulus, generically labelled 8 in the accompanying drawings.

Each emitter 3A, 3B, 4A, 4B, 5A, 5B is connected to a computerised control unit 9, inside a suitable box-shaped body, at a respective output 10, 11 and 12, whilst there is a free output 13 for any further emitters which are not illustrated.

The unit 9 has a memory 9a and a program for operating the device 1, located in the memory 9a, and is designed to generate the above-mentioned stimulation signals.

The device 1 also comprises means 14 suitable for modulating the signal emitted. These modulating means may be of any known type and are not described in further detail.

Modulation of the single signal or the sequence of signals emitted is preferably controlled by a suitable operating program for the device disclosed, located in the computerised unit 9.

For example, the single signals may have a predetermined waveform and may be subject to frequency, duration and intensity modulation.

Moreover, the sequences of signals, that is to say, successive emissions of single stimulation signals, may be suitably modulated, in particular according to a predetermined sequence.

According to a preferred procedure, the modulating means 14 are operated manually using suitable regulating means consisting of a set of push-buttons 15, in particular four in the embodiment illustrated by way of example, which, preferably, provide controls interacting with the unit 9.

The device 1 also comprises stimulation signal control means 16, it being possible for the control means 16 to be operated automatically, according to methods described in detail below, in particular to generate a sequence of signals depending on the state of the user.

The emitters 3A, 3B, 4A, 4B, 5A, 5B of each pair can transmit the respective signal sequentially, that is to say, in such a way that they alternate.

In another mode, the emitters 3A, 3B, 4A, 4B, 5A, 5B of each pair can transmit the respective signal simultaneously or synchronously.

Similarly, the signals may be of the type with a single pulse or with a succession or train of pulses, which may have constant or variable intensity.

In the latter case, the intensity may vary randomly or periodically, or the sequence may vary in a predetermined way.

The sequence of signals emitted may be set up to be variable, preferably variable in a predetermined way, or maintaining the same regularity.

In practice, a series of sequences of signals, each sequence emitting signals with respective characteristics and having predetermined time intervals may be sent.

For example, it would be possible to have a series of sequences in which there is a first step or sequence of emission of a signal with predetermined intervals, followed by a second step or sequence with emission, again at predetermined intervals of another signal, followed by a third step or sequence with emission, again at predetermined intervals of another signal and so on.

It should also be noticed that the separate emitters 3A, 3B, 4A, 4B, 5A, 5B of each pair can emit signals which are the same, or signals which are different, each having its own characteristics. Signals of different types, acoustic, tactile or visual and other types may also reach the user 2 simultaneously or in a predetermined sequence.

Said signals are preferably sent sequentially on the right-hand side 2a of the user's body and then on the left-hand side 2b of the same user's body, and so on. Multiple stimulations are produced, for example tactile, visual and acoustic, which alternately stimulate the right-hand side and the left-hand side of the user. Obviously, other methods for stimulating the user are also applicable.

As indicated, the signals and/or sequences of them may be generated instantly by the unit 9 and the modulating means 14 and, alternatively, may be saved, using suitable save instructions, in the above-mentioned memory 9a and suitably retrieved from it using one or more of the push-buttons 15, and emitted, by the emitters 3A, 3B, 4A, 4B, 5A, 5B, 6, 7 and 8.

Looking at the construction architecture of the various emitters in greater detail, it should be noticed that the emitters 3A and 3B comprise vibration means 17 of the known type, transducing the stimulation signal into a mechanical vibration. These vibrating elements comprise a vibrating surface 18, in contact with supporting means 17, 19 suitable for bringing it into contact with a corresponding part of the user's 2 body.

The emitters 3A, 3B also comprise a grip body or contact 20, designed to be held by the user 2 or placed in contact with the user, so that the vibrating surface 18 is in contact with the user's body.

The emitters 4A, 4B comprise an extended supporting body 21, between 2 and 2000 cm long, preferably between 4 cm and 60 cm, which supports, at its ends 21a and 21b a first and a second light source 22 and 23. In other words, the emitters 4A and 4B consist of the light sources 22 and 23 at the ends 21a and 21b of the body 21.

The emitters 5A and 5B have connecting and supporting means 24 consisting, in the embodiment illustrated, of headphones which fit on the user's 2 head.

As already indicated, the device 1 may operate with feedback of information about the state of the user 2.

In this case, there are means 25, comprising sensors or feedback instruments, for detecting a signal corresponding to a user 2 parameter, acquired by the device 1 by means of an acquisition and measuring unit 26 and transmitted to the computerised control unit 9, controlled by a suitable management or operating program.

The means 16 for automatically controlling or regulating the signals emitted modulate the stimulation signals according to the signal corresponding to the user 2 parameter or parameters. In this way, the signal or the specific sequence of signals can be modified to better adapt it to the user's condition.

By means of a communication unit 27, the device 1 may also interface with external processing means 32, if the operating program of the device disclosed has suitable means or instructions for the management of such data exchange.

In the preferred embodiment illustrated, the device 1 also has a display 28 for assisting with management of the device 1.

Therefore, the operating program of the device disclosed may comprise means or instructions for performing one or more predetermined sequences of signals saved in the memory.

In particular, the operating program comprises means or instructions for selecting one or more predetermined operating sequences saved in the memory.

The device may also have means for reading an external memory. The external memory may be a magnetic, optical, semiconductor or other type of memory.

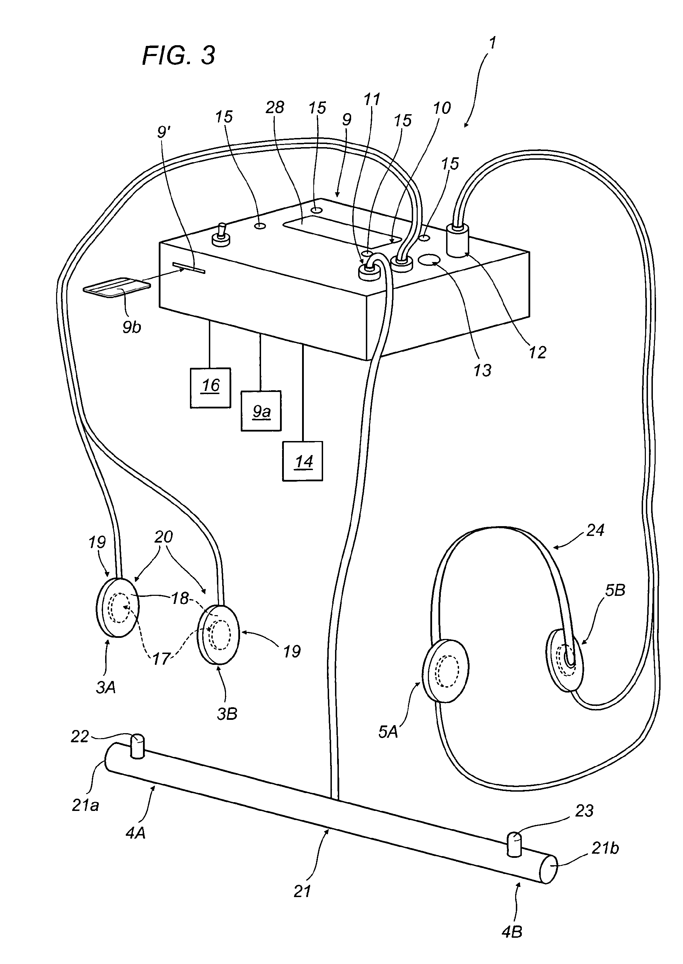

The external or removable memory is preferably supported by a card 9b, for example a plastic card with a magnetic strip, or equipped with a semiconductor device, on which a customised procedure may be saved. The card 9b can be inserted in a suitable reader - writer, belonging to the device, whose device access slot 9' is illustrated in Figure 3, for reading and, if necessary, rewriting. It can then be removed from the device and kept and reused.

The device operating program may have means or instructions which allow the performance of only a predetermined series of stimulation sequences. This prevents the device from emitting sequences of signals that are unwanted and/or potentially harmful for the specific user.

Advantageously, the device operating program has means or instructions designed to perform a customised procedure.

In particular, said means or instructions designed to perform a customised procedure are able to detect a therapist identification code, belonging to the person who has established the sequence to be performed, and a subject or user identification code.

Moreover, said means or instructions designed to perform a customised procedure are able to detect identification parameters of the specific stimulation sequence to be performed.

The means or instructions designed to perform a customised procedure can also detect the number of stimulation sessions, or series of signal sequences which can be performed daily and the maximum number of stimulation sessions to be performed by the subject or user.

Said data or parameters identifying or specific to a customised operating procedure can be saved on the card, or another similar medium, or can be saved in the device memory and can be suitably retrieved following a specific identification command.

In particular, the present operating program has means or instructions which, if the maximum number of stimulation sequences has been reached, are designed to stop any further operating sequences from being performed.

The present invention also relates to an apparatus 29 consisting of a device 1 as described above connected to a local viewing and programming unit or personal computer 30, having a keyboard and monitor for interfacing with a generic user.

The computer has both a program for management of viewing and a program for setting the device 1 operating parameters.

In practice, the device 1, which may be managed by an operator 31, stimulates the user 2 in various ways. In particular, if feedback is used, the stimulation signals are generated automatically by the device, depending on the state and reactions of the user.

The present invention brings important advantages. In particular the device 1 is very flexible in generating stimulation signals, and, since it can be provided with feedback in the form of information about the state of the user, its operation is highly and automatically customisable depending on the user treated.

Thus, in practice, the operator has a wide range of treatment possibilities available to him, which he can use to achieve the desired results for the user.

The operator can manually regulate the signals according to his perception of user requirements, access a series of predetermined sequences, already in the memory. He can also modify the sequences, to "calibrate them" to the user's specific characteristics and conditions. The customised sequences can be saved and reused for the same user, during a subsequent session, or for a different user with similar characteristics.

The device disclosed may also be used to achieve health and therapeutic effects such as inducing a simple state of relaxation, boosting memory, concentration and tolerance of pain, for learning, to facilitate new motor or sensory schemes, controlling repetitive and obsessive thoughts, post traumatic stress disorders, and even for psycho-neurosensory re-education and other purposes.

It will be understood that the invention can be modified and adapted in several ways without thereby departing from the scope of the inventive concept. Moreover, all the details of the invention may be substituted by technically equivalent elements.

## Claims

1. A device for neurosensory stimulation of a user (2); comprising emitter means (3A, 3B, 4A, 4B, 5A, 5B, 6, 7, 8) for a neurosensory stimulation signal which can be perceived by a user (2), **characterised in that** there are means (14) for modulating the signal emitted.

2. The device according to claim 1 or according to the preamble to claim 1, **characterised in that** the emitter means (3, 4, 5, 6, 7, 8) comprise a first emitter element (3A, 4A, 5A) for a stimulation signal and a second emitter element (3B, 4B, 5B) for a stimulation signal.

3. The device according to claim 2, **characterised in that** the first and the second emitter elements (3A, 3B, 4A, 4B, 5A, 5B) for a stimulation signal emit a first and a second signal sequentially, that is, one after another, or synchronously, that is, simultaneously.

4. The device according to any of the foregoing claims or according to the preamble to claim 1, **characterised in that** the signal emitted is in the form of a single pulse.

5. The device according to any of the foregoing claims or according to the preamble to claim 1, **characterised in that** the signal emitted is in the form of a train or succession of pulses.

6. The device according to claim 5, **characterised in that** said pulses have a constant intensity.

7. The device according to claim 5, **characterised in that** said pulses have a variable intensity.

8. The device according to claim 7, **characterised in that** said pulses have a randomly variable intensity.

9. The device according to any of the foregoing claims from 5 to 8, **characterised in that** the succession of pulses has a predetermined rhythm.

10. The device according to any of the foregoing claims from 5 to 8, **characterised in that** the succession of signals emitted is variable in a predetermined way.

11. The device according to any of the foregoing claims from 5 to 8, **characterised in that** the succession of pulses in the signal has a predetermined waveform.

12. The device according to any of the foregoing claims or according to the preamble to claim 1, **characterised in that** the signal or sequence of signals emitted is variable depending on the state of the user.

13. The device according to any of the foregoing claims from 2 to 12, **characterised in that** the first and second emitter elements (3A, 3B, 4A, 4B, 5A, 5B) for a corresponding stimulation signal emit a first and a second signal which are the same as one another.

14. The device according to any of the foregoing claims from 2 to 13, **characterised in that** the first and second emitter elements (3A, 3B, 4A, 4B, 5A, 5B) for a corresponding stimulation signal emit a first and a second signal which are different to one another.

15. The device according to any of the foregoing claims, **characterised in that** the means (14) for modulating the signal emitted modulate the frequency of the signal emitted.

16. The device according to any of the foregoing claims, **characterised in that** the means (14) for modulating the signal emitted modulate the duration of the signal emitted.

17. The device according to any of the foregoing claims, **characterised in that** the means (14) for modulating the signal emitted modulate the intensity of the signal emitted.

18. The device according to any of the foregoing claims, **characterised in that** the means (14) for modulating the signal emitted modulate the waveform of the signal.

19. The device according to any of the foregoing claims, **characterised in that** the means (14) for modulating the signal emitted modulate the rhythm of the signal pulses.

20. The device according to any of the foregoing claims or according to the preamble to claim 1,
**characterised in that** the signal emitted is a signal which the user can perceive by touch.

21. The device according to claim 20, **characterised in that** said signal which can be perceived by the user by touch is in the form of a mechanical vibration.

22. The device according to claim 21, **characterised in that** the emitter means comprise at least one stimulating element (3A) having a vibrating surface (18) and supporting means (19) suitable for putting the vibrating surface (18) in contact with a corresponding part of the body of the user (2).

23. The device according to claim 22, **characterised in that** the emitter means comprise at least a second stimulating element (3B) having a vibrating surface (18) and supporting means (19) suitable for putting the vibrating surface (18) in contact with a corresponding part of the body of the user (2).

24. The device according to claim 22 or 23,
**characterised in that** the stimulating element (3A, 3B) comprises a body (20) which can be gripped by the user (2) and vibrating means (17) between the grip body (20) and the stimulating surface.

25. The device according to any of the foregoing claims or according to the preamble to claim 1, **characterised in that** the signal emitted is a signal which the user can perceive visually.

26. The device according to claim 25, **characterised in that** the emitter means comprise a first and a second light source (22, 23) located at the ends (21a, 21b) of an extended supporting body (21).

27. The device according to claim 26, **characterised in that** the extended supporting body (21) is between 2 and 2000 cm long.

28. The device according to any of the foregoing claims or according to the preamble to claim 1, **characterised in that** the signal emitted is a signal which the user (2) can perceive acoustically.

29. The device according to claim 28, **characterised in that** the emitter means comprise a first and a second acoustic emitter element (5A, 5B) and means (24) for connecting the first and second acoustic emitter elements to the ears of the user (2).

30. The device according to any of the foregoing claims or according to the preamble to claim 1, **characterised in that** the signal emitted is an electrical signal.

31. The device according to any of the foregoing claims or according to the preamble to claim 1, **characterised in that** the signal emitted is a chemical signal.

32. The device according to any of the foregoing claims, **characterised in that** the means (14) for modulating the signal emitted comprise regulating or control means (15) which can be operated manually.

33. The device according to claim 32, **characterised in that** the regulating means which can be operated manually comprise at least a first and a second push-button (15).

34. The device according to any of the foregoing claims or according to the preamble to claim 1, **characterised in that** there are means (25) for detecting a signal corresponding to a user (2) state parameter.

35. The device according to any of the foregoing claims, **characterised in that** the means (14) for modulating the signal emitted comprise control means (16), which cause the automatic emission of a corresponding signal, in particular in response to the signal detected.

36. The device according to any of the foregoing claims or according to the preamble to claim 1, comprising processing means, having a CPU (9), a memory (9a) and input and output means (10, 11, 12) and an operating program saved in the memory (9a).

37. The device according to claim 36, comprising an operating program defining means (27) or instructions designed to manage an exchange of data with processing means (32) external to the device.

38. The device according to claim 36 or 37, comprising a display or monitor (28).

39. The device according to any of the foregoing claims from 36 to 38, **characterised in that** the operating program comprises means or instructions for saving predetermined sequences of signals in a predetermined area of the memory.

40. The device according to any of the foregoing claims, **characterised in that** the modulating means modulate the succession of single stimulation signals, in particular according to a predetermined sequence.

41. The device according to any of the foregoing claims, **characterised in that** it has means for reading an external memory.

42. The device according to any of the foregoing claims, **characterised in that** it has means for writing to an external memory.

43. The device according to any of the foregoing claims, comprising a removable memory for saving a customised procedure.

44. The device according to any of the foregoing claims, **characterised in that** there are means which allow only predetermined stimulation sequences to be performed.

45. The device according to any of the foregoing claims, **characterised in that** there are means designed to perform a customised procedure.

46. The device according to claim 45, **characterised in that** the means designed to perform a customised procedure detect a therapist identification code.

47. The device according to claim 45 or 46,
**characterised in that** the means designed to perform a customised procedure detect a user identification code.

48. The device according to any of the foregoing claims, **characterised in that** the means designed to perform a customised procedure detect the identification parameters of the specific stimulation sequence.

49. The device according to any of the foregoing claims from 45 to 48, **characterised in that** the means designed to perform a customised procedure detect the daily number of stimulation sessions.

50. The device according to any of the foregoing claims from 45 to 49, **characterised in that** the means designed to perform a customised procedure detect the maximum number of stimulation sessions to be performed by the user.

51. The device according to any of the foregoing claims, **characterised in that** the visual, tactile and acoustic stimulation signals reach the user (2) simultaneously or alternately, on the right-hand side and on the left-hand side of the user (2).

52. The device according to any of the foregoing claims, **characterised in that** a series of sequences of signals is sent.

53. The device according to claim 52, **characterised in that** the series of sequences includes at least a first sequence with emission of a signal at predetermined intervals and a second sequence with emission of another signal, again at predetermined intervals.

54. The device according to any of the foregoing claims, **characterised in that** the emitters (3A, 3B, 4A, 4B, 5A, 5B) in each pair can emit signals which are the same as one another or different to one another.

55. An apparatus for neurosensory stimulation of a user, **characterised in that** it comprises a device (1) according to any of the foregoing claims and processing means (32) external to the device.

56. The apparatus according to claim 55, **characterised in that** the external processing means are in the form of a PC (30) having a monitor and a control keyboard.

57. The apparatus according to claim 56, **characterised in that** the PC comprises a program for management of stimulating device (1) operating parameter viewing.

58. The apparatus according to claim 56 or 57, **characterised in that** the PC (30) comprises a program for setting the stimulating device (1) operating parameters.
